(19) Europäisches Patentamt / European Patent Office / Office européen des brevets

(11) **EP 1 639 903 B1**

(12) **EUROPEAN PATENT SPECIFICATION**

(45) Date of publication and mention
of the grant of the patent:
**17.12.2008 Bulletin 2008/51**

(51) Int Cl.:
*A23L 1/305* (2006.01)  *A23L 1/308* (2006.01)
*A23L 1/30* (2006.01)  *A61K 31/736* (2006.01)
*A61K 36/00* (2006.01)  *A61K 38/16* (2006.01)
*A23K 1/16* (2006.01)

(21) Application number: **04746157.9**

(22) Date of filing: **15.06.2004**

(86) International application number:
**PCT/JP2004/008684**

(87) International publication number:
**WO 2004/110174 (23.12.2004 Gazette 2004/52)**

(54) **COMPOSITION AND FOODS FOR LOWERING GLYCEMIC INDEX**

ZUSAMMENSETZUNG UND NAHRUNGSMITTEL ZUR ERNIEDRIGUNG DES GLYCEMISCHEN
INDEX

COMPOSITION ET ALIMENTS POUR ABAISSEMENT DE L'INDICE GLYCEMIQUE

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **16.06.2003 JP 2003171214
12.12.2003 JP 2003415510
27.02.2004 JP 2004054067**

(43) Date of publication of application:
**29.03.2006 Bulletin 2006/13**

(73) Proprietor: **TAIYO KAGAKU Co., LTD.
Yokkaichi-shi
Mie 510-0825 (JP)**

(72) Inventors:
 • **YOKAWA, Takeo,
Taiyo Kagaku Co., Ltd.
Yokkaichi-shi,
Mie 510-0825 (JP)**
 • **ISHIHARA, Noriyuki,
Taiyo Kagaku Co., Ltd.
Yokkaichi-shi,
Mie 510-0825 (JP)**
 • **AOYAMA, Nobuhiko,
/Taiyo Kagaku Co., Ltd.
Yokkaichi-shi,
Mie 510-0825 (JP)**
 • **JUNEJA, Lekh Raj,
/Taiyo Kagaku Co., Ltd.
Yokkaichi-shi,
Mie 510-0825 (JP)**

(74) Representative: **HOFFMANN EITLE
Patent- und Rechtsanwälte
Arabellastrasse 4
81925 München (DE)**

(56) References cited:
 JP-A- 1 020 063   JP-A- 5 117 156
 JP-A- 6 247 865   JP-A- 9 252 792
 JP-A- 2003 516 757

 • CHIU SS, TRACK NS: "Guar by-product improves
carbohydrate tolerance in rats" METABOLISM,
vol. 34, no. 5, May 1985 (1985-05), pages 481-485,
XP002415531

EP 1 639 903 B1

**Description**

TECHNICAL FIELD

The present invention relates to a composition for lowering a glycemic index and a low-glycemic index food.

BACKGROUND ART

According to the survey conducted in 1997 by today's Ministry of Health, Labor and Welfare, the number of people who are strongly suspected to be diabetic patient is estimated to be about 6.9 million, and the number of people who are undeniable to be diabetic patient is said to reach about 6.8 million. The number of diabetic patients including potential diabetic patients is said to reach about 20 million. The reasons for the increase in the number of diabetic patients are divided into genetic factors and those originated from living habits such as "obesity, hyperphagia, lack of exercise, and irregular lifestyle", and mainly the number of the diabetic patients caused by the latter reasons has been increasing. The mechanism of the onset is that in the case of hyposecretion of insulin associated with postprandial hyperglycemia, or when insulin is secreted but shows resistance and does not work as a hormone, disordered saccharometabolism takes place, which results in diabetes. Therefore, diet regimen which limits the carbohydrates from meal or pharmacotherapy such as a sugar absorption inhibitor is used for metabolizing sugar with limited insulin.

In accordance with the social climate as described above, as a result of recent progress in development of artificial pancreas or widespread use of self monitoring of blood glucose, normalization of blood glucose over a period of 24 hours has been revealed to be essential for the onset and development of diabetic angiopathy, and the concept of a glycemic index (hereinafter referred to as GI value in some cases) of foods has been introduced by Jenkins et al. in 1982 (see, for example, Jenkins DJ, Ghafari H, Wolever TM, Taylor RH, Jenkins AL, Barker HM, Fielden H, Bowling AC: Relationship between rate of digestion of foods and post-prandial glycaemia, Diabetologia, Vol. 22, 450-455 (1982)).

A glycemic index of a food refers to an index showing the magnitude of the peak of blood glucose level which elevates when the food is ingested. Generally, the glycemic index is obtained by indexation of change of blood glucose level after ingesting various foods as compared with the blood glucose level after ingesting glucose, when the index of any of glucose, polished rice and bread is defined as 100. The lower this index is, the smaller the amount of insulin that functions to lower blood glucose level secreted. When the glycemic index is high, since insulin is secreted in excess, beta-cells of islets of Langerhans of pancreas become strained, which leads to onset of diabetes. Briefly, it is considered that the glycemic index of a food to be ingested is controlled, thereby reducing the load on pancreas whereby the onset of diabetes can be prevented. Since it has been found that sudden elevation in postprandial blood glucose level increases the load on the secretion of insulin from beta-cells of islets of Langerhans of pancreas in diabetic patients, this index has been already used in a standard dietary direction or the like for diabetic patients in Australia or the likes.

Some attempts to control a glycemic index have been made so far. Heaton et al. has reported that a glycemic index is controlled by difference in particle sizes of wheat, maize, and oat (see, for example, Heaton KW, Marcus SN, Emmett PM, Bolton CH: Particle size of wheat, maize, and oat test meals: effects on plasma glucose and insulin responses and on the rate of starch digestion in vitro, Am. J. Clin. Nutr., Vol. 47, 675-682 (1988)). Moreover, it has been known that a glycemic index of a food is lower when ingesting boiled rice than ingesting powdered rice and lower when ingesting an apple in whole than ingesting a "pureed" apple (see, for example, Kunihiro Doi and Keisuke Tsuji Eds., Shokumotsu Sen-i (Dietary Fiber), p.412-420 (Asakura-shoten, Tokyo, 1997)). Besides, methods utilizing a polysaccharide having gel formation ability, such as guar gum, pectin, or glucomannan have been known. These are the methods for lowering the GI value of a food, in which extension of endogastric residence time of glucose due to gel formation (see, for example, "Kagaku to Seibutsu (Chemistry and Biology), " Vol. 18, p95-105, 1980).

These attempts are, however, methods of controlling the GI value utilizing the difference in digestion and absorption depending upon the forms of foods, so that it is necessary to process foods to utilize. Therefore, these methods have limitations of the cost required for processing or the foods which can be used. There is also a problem that the forms of the foods are limited, and that the polysaccharide having gel formation ability, such as guar gum or pectin, is very highly viscous, thereby making it difficult to add the polysaccharide to an ordinary food or to process the polysaccharide.

Now, methods for inhibiting a drastic elevation in blood glucose level using a hydrolyzed guar gum as an active principle are reported (see, for example, publication JP 05 117 156 on pages 1 to 4). Generally, it is believed that the inhibitory effect on elevation in blood glucose level is exhibited by guar gum, which is a kind of a natural polysaccharide in the *Cyamopsis tetragonolobus* bean components. On the other hand, it has not so far been reported that inhibitory effect on elevation in blood glucose level is exhibited or enhanced by using a *Cyamopsis tetragonolobus* bean proteins. From Chiu SS, Track NS, Metabolism, Vol. 34, No. 5, May 1985, pp 481-485, a guar byproduct is known, which improves carbohydrate tolerance in rats. Said article does not describe a degradation of galactomannan.

From JP 05 117156 A, a preventive and therapeutic agent for diseases following hyperglycemia is known which comprises guar gum hydrolysate as an active ingredient, but does not refer to bean proteins.

DISCLOSURE OF INVENTION

An object of the present invention is to provide an effective and safe composition for lowering a glycemic index (GI value) of a food or feed which is easily applicable to a food or feed, and a low-glycemic index food or feed.
Concretely, the present invention relates to:

[1] a composition for lowering a glycemic index (GI value) of a food or feed, comprising a *Cyamopsis tetragonolobus* bean protein and a degraded (=hydrolysed) galactomannan;
[2] the composition according to the above [1], wherein the weight ratio of the *Cyamopsis tetragonolobus* bean protein to the degraded galactomannan (*Cyamopsis tetragonolobus* bean protein / degraded galactomannan) is from 1/99 to 1/5;
[3] the composition according to the above [1] or [2], wherein the average molecular weight of the degraded galactomannan is from 2000 to 100000;
[4] the composition according to any one of the above [1] to [3], wherein the viscosity of a 0.5(w/v)% aqueous solution of the degraded galactomannan is 50 mPa•s or less at 25°C when determined with a B Type Viscometer;
[5] a food or feed comprising the composition as defined in any one of the above [1] to [4];
[6] use of the low-glycemic index food as defined in any one of the above [1]-[5] for the manufacture of a food for specified health use or a food for a diabetic patient.

The composition for lowering a glycemic index (GI value) provided by the present invention comprises the components used as foods, and do not have toxicity in rerum natura. The degraded galactomannan used is water-soluble and has low viscosity, differing from guar gum or pectin, so that the degraded galactomannan is a safe and easily utilizable material that does not cause endogastric accumulation. Therefore, the composition is suitable for processing, taking or the like, and the composition is also easily applicable to a food or feed, whereby the GI value of various foods or feed can be lowered effectively and safely. Further, a low-GI value food or feed can be easily obtained by adding the composition to an ordinary food or feed. Moreover, a low-glycemic index food comprising specified carbohydrate-related components is described for reference. The composition, food and feed of the present invention can significantly contribute to reduce the load on beta-cells of islets of Langerhans of pancreas for insulin secretion resulting from ingestion of a food and the like, and therefore can be effectively used for prevention of onset of diabetes or amelioration of diabetes

BEST MODE FOR CARRYING OUT THE INVENTION

Generally, a GI value is a term used for foods for human. However, the term may be used herein for feed as well as foods. The phrase "GI value for a food or feed" as used herein refers to numerically expressed change in blood glucose level upon ingestion of a food or feed in an amount 50 g calculated as the carbohydrates, wherein the elevation or the lowering in the blood glucose level in the case where 50 g of glucose was ingested by an individual is defined as 100. Concretely, the "GI value for a food or feed" can be obtained from the following formula:

$$\text{GI value} = [(\text{Area below the curve of blood glucose level until 2 or 3 hours after ingesting a food or feed (amount calculated as carbohydrates: 50 g})) / (\text{Area below the curve of blood glucose level until 2 or 3 hours after ingesting glucose (50 g}))] \times 100$$

In the formula, the "area below the curve of blood glucose level" is obtained by drawing the graph of the blood glucose level of an individual to a time period after ingestion of a food or feed, or glucose by an individual, and calculating the area below the curve of blood glucose level until 2 or 3 hours after ingestion. Here, the time after ingesting a food or feed and the time after ingesting glucose are set to have the same conditions. The glucose serving as a standard can be replaced with bread as long as the bread weighs 50 g calculated as carbohydrates.
The phrase "lowering (of) a GI value" as used herein means that the GI value of the food or feed is lowered when the composition for lowering a glycemic index of the present invention is applied to the food or feed, as compared with the GI value of a food or feed before application of the composition, to which the composition is to be applied (for example, ingested concomitantly with the composition).

The low-glycemic index food (including feed) as used herein refers to a food of which GI value is lowered as compared to that of a conventional food corresponding to the food (referred to herein as a corresponding food) upon the determination in an identical individual. The lowering ratio of a GI value can be obtained from the following formula:

$$\text{Lowering ratio of GI value (\%)} = [1 - (\text{GI value of food to be judged}) /$$
$$(\text{GI value of the corresponding food})] \times 100$$

wherein a food which is to be judged whether or not to be a low-glycemic index food is expressed as a food to be judged. The lowering ratio of a GI value is preferably 10%, and more preferably 20%.

The lowering of a GI value may be found at a point 2 or 3 hours after the ingestion of the food or feed.

The GI value cannot be flatly defined because the lowering of GI values varies among individuals or depending on the determination conditions. The GI value of the low-glycemic index food of the present invention is preferably 70 or less, and more preferably 60 or less.

Each of the composition for lowering a GI value and the low-GI value food of the present invention will be separately explained hereinafter. Each component of the composition and the food of the present invention described below can be appropriately used alone or in a mixture of two or more kinds.

[1] Composition for Lowering a Glycemic Index

A great feature of the composition for lowering a GI value of a food or feed of the present invention (hereinafter referred to as a composition) resides in that the composition comprises a *Cyamopsis tetragonolobus* bean protein and a degraded galactomannan.

Surprisingly, the present inventors have found for the first time that especially use of a *Cyamopsis tetragonolobus* bean protein, which has not yet been reported to have an inhibitory effect on elevation in blood glucose level, concomitantly with a degraded galactomannan (for example, the above-mentioned hydrolyzed guar gum) known for the effect and ingestion of these together with a food can synergistically remarkably lower the GI value of the food as compared to any of the substances which are known for their inhibitory effects on elevation in blood glucose level. Although the details of the mechanism for exhibiting the synergistic effects of a *Cyamopsis tetragonolobus* bean protein and a degraded galactomannan in lowering a GI value of a food are unclear, it is presumed that a *Cyamopsis tetragonolobus* bean protein has some influences on the inhibitory effect on elevation in blood glucose level by a degraded galactomannan, thereby synergistically improving the effects.

The *Cyamopsis tetragonolobus* bean protein contained in the composition of the present invention includes all sorts of proteins which can be extracted from *Cyamopsis tetragonolobus* beans. The protein also includes a protein which is not extracted directly from *Cyamopsis tetragonolobus* beans. For example, when the gene sequence of a *Cyamopsis tetragonolobus* bean protein is known, those proteins obtained by genetic engineering on the basis of the sequence according to known methods are also included. Further, as long as the desired effects of the present invention can be exhibited, the protein can be a variant of a *Cyamopsis tetragonolobus* bean protein containing a mutation in one or more amino acids. The protein as referred to herein is a peptide having at least 3 amino acid residues. Moreover, the *Cyamopsis tetragonolobus* bean protein may be composed of a single protein, or a mixture of two or more kinds of proteins.

The *Cyamopsis tetragonolobus* bean protein of the present invention can be easily obtained, for example, by treating *Cyamopsis tetragonolobus* beans by a combined method of known purifying means for proteins. For example, the method is preferably, but not particularly limited to, the following method.

Using *Cyamopsis tetragonolobus* beans as a raw material, soluble components are eluted with an aqueous solvent of pH from 7 to 9, such as water or an aqueous alkali solution. The *Cyamopsis tetragonolobus* beans may be pulverized before or after subjecting the *Cyamopsis tetragonolobus* beans to the solvent as desired. The mixture after the elution of soluble components is subjected to centrifugation, and thereafter insoluble components are removed in the form of precipitates. An acid is added to the supernatant to adjust the pH to about 4.5, and the protein is allowed to precipitate. The mixture is subjected to centrifugation again, to separate the mixture into a supernatant and precipitates. The procedures of adding water to the precipitates to disperse the precipitate, and centrifuging the dispersion are repeated, to wash the precipitates. An aqueous solvent of pH of about 7, such as water or an aqueous alkali solution, is added to the precipitates, to neutralize and dissolve the precipitates. The resulting solution is spray-dried as desired, to give a *Cyamopsis tetragonolobus* bean protein in the form of powder. Here, as the above-mentioned acid, for example, hydrochloric acid, sulfuric acid, citric acid, acetic acid, phytic acid or the like is used, and as the above-mentioned alkali, for example, sodium hydroxide, potassium hydroxide or the like is used.

By the procedures described above, the *Cyamopsis tetragonolobus* beans are collected in an amount of from 30 to 40% by weight as a protein. The protein content in the resulting *Cyamopsis tetragonolobus* bean protein is usually 90% by weight or more, a part of which contains impurities. Here, it has been confirmed that the impurities do not include a guar gum component.

As the *Cyamopsis tetragonolobus* bean protein, a commercially available product can be used if the product is available.

Furthermore, for example, these *Cyamopsis tetragonolobus* bean proteins which are appropriately degraded with an enzyme protease (manufactured by Novo Nordisk Bioindustry) or the like can be used, as long as the exhibition of the desired effects of the present invention can be obtained. The resulting degraded matters can be used alone or together with a product that has not undergone degradation.

On the other hand, the degraded galactomannan is obtained by hydrolyzing any raw material containing galactomannan as a chief component by a known method to make the molecular weight low. Such raw material includes natural mucopolysaccharides such as guar gum, locust bean gum, tara gum, cassia gum, and sesbania gum. Especially, from the viewpoint of ease in preparation by hydrolysis, the raw material of the degraded galactomannan is preferably guar gum, locust bean gum and sesbania gum, and more preferably guar gum and locust bean gum. Each of these raw materials can be used alone or in a mixture of two or more kinds.

Methods of hydrolysis include, but not particularly limited to, enzymolysis, acidolysis and the like. From the viewpoint that the molecular weight of the degraded products can be easily evenly sized, enzymolysis is preferable.

The enzyme used for enzymolysis is particularly not limited, and may be commercially available products, those derived from natural products, or those obtained by known recombinant techniques, as long as the enzyme is capable of hydrolyzing mannose straight chain. From the viewpoint of enhancing the degradation efficiency, the enzyme is preferably β-mannanase derived from a bacteria belonging to *Aspergillus, Rhizopus* or the like.

The conditions for the enzymolysis of the above-mentioned raw materials cannot be described flatly because they vary depending on the enzyme used. Ordinary conditions include, for example, the conditions wherein a reaction is carried out at 10° to 80°C for about 1 to about 75 hours in the presence of the enzyme in an amount of from 0.1 to 20 parts by weight based on 100 parts by weight of the raw material in a buffer suitable for the enzyme used.

The conditions for acidolysis include, but not particularly limited to, for example, the conditions wherein a reaction is carried out at 90° to 100°C for 1 to 40 hours in any solvent having a pH of from 1 to 4.

By the procedures described above, the desired degraded galactomannan can be obtained. The resulting degraded product can be used directly, or can be used after washed with water or the like as desired. Further, commercially available products can be used. The commercially available products include, for example, "SUNFIBER (trade name)" [manufactured by Taiyo Kagaku Co., Ltd.], "Fiberon (trade name)" [manufactured by DAINIPPON PHARMACEUTICAL CO., LTD.] and the like.

The degraded galactomannan used in the present invention has an average molecular weight of preferably from 2000 to 100000, more preferably from 8000 to 50000, and even more preferably from 15000 to 25000, from the viewpoint of excellent exhibition of the desired effects and usefulness.

The average molecular weight can be obtained, for example, by subjecting the degraded galactomannan to high-performance liquid chromatography [column manufactured by YMC Co., Ltd.: YMC-Pack Diol-120] using polyethylene glycols (molecular weights: 2000, 20000 and 100000) as molecular weight markers to obtain the molecular weight distribution, numerically expressing the molecular weight distribution by applying the molecular weight distribution to the calibration curve obtained by the molecular weight markers, and averaging the resulting values.

From the same viewpoint as in the case of the average molecular weight, the degraded product in the form of an aqueous 0.5(w/v)% solution has a viscosity of preferably 50 mPa•s or less, more preferably 30 mPa•s or less, and even more preferably 10 mPa•s or less when determined with a B Type Viscometer at 25°C. Here, the determination with the B Type Viscometer described herein is usually carried out using a rotor No. 1, under the conditions of a rotational speed of 20 rpm.

As the degraded galactomannan of the present invention, those falling within the above-mentioned preferable ranges for both the average molecular weight and the viscosity mentioned above are especially preferable.

In addition, the composition of the present invention may contain other components as long as the exhibition of the desired effects of the present invention would not be inhibited. The components include, but not particularly limited to, for example, water, such as tap water, distilled water, and ion-exchanged water; proteins other than the *Cyamopsis tetragonolobus* bean protein; amino acids; peptides; dietary fibers; tea extracts such as polyphenols; and the like. Among them, dietary fiber is preferable.

The composition of the present invention comprises a *Cyamopsis tetragonolobus* bean protein and a degraded galactomannan, or further comprises other components as desired as described above. The content of the *Cyamopsis*

*tetragonolobus* bean protein and the degraded galactomannan in the composition is preferably from 0.1 to 100% by weight. The other components may be appropriately contained within a range that does not inhibit the desired effects of the present invention.

Especially, the combination of the *Cyamopsis tetragonolobus* bean protein and the degraded galactomannan and the ratio of both of these components in the composition are significant in the composition of the present invention for the exhibition of the effects. The weight ratio of the *Cyamopsis tetragonolobus* bean protein to the degraded galactomannan (*Cyamopsis tetragonolobus* bean protein / degraded galactomannan) is preferably from 1 / 99 to 1 / 5, more preferably from 1 / 20 to 1 / 4, and even more preferably from 1 / 15 to 1 / 3. When the weight ratio is within the above range, the desired effects are satisfactorily exhibited, so that there is no problem on its application to various foods, making it favorable.

Furthermore, the form of the composition of the present invention is not limited to, and can be, for example, powder, tablet, emulsion, solution and the like, as far as the exhibition of the desired effects of the present invention is not inhibited.

The composition of the present invention can be prepared by mixing each of these components according to a known method (for example, a method used in the food industry). During mixing, the mixture can be appropriately formed into a desired form.

As described above, the composition of the present invention can be obtained. The composition is effective in lowering a GI value of a food or feed for any individual (animal), especially for a mammal, even more especially for a food for human. Moreover, in recent years, the development of diseases dependent on living habit has become a social concern among household pets and the like. The composition will probably be effective in lowering a GI value of feed for the pets and the like.

The effects of the composition of the present invention are exhibited by ingesting the composition together with the food or feed of which GI value is desired to be lowered. Therefore, it is preferable to ingest the composition of the present invention so that the composition of the present invention is present in the digestive tract of the above-mentioned individual together with the food before or after the ingestion of the food or feed, or during the ingestion, or at least when the digestion of the food or feed starts. Usually, the composition may be taken when the food or feed is taken in the form prepared, for example, by dissolving (or dispersing) the composition (powder) of the present invention in water.

The amount of the composition of the present invention to be ingested is not particularly limited. It is preferable that the weight ratio of the carbohydrates in a food or feed to the composition of the present invention (calculated as the total amount of a *Cyamopsis tetragonolobus* bean protein and a degraded galactomannan) (carbohydrates in the food or feed / composition of the present invention) is preferably from 50 / 1 to 1 / 10, more preferably from 40 / 1 to 1 / 5, and even more preferably from 20 / 1 to 1 / 1, from the viewpoint of obtaining a satisfactory effect of lowering a GI value of the food or feed.

Moreover, as one embodiment of the present invention, a food or feed comprising the composition of the present invention is provided. The food or feed is one embodiment of the low-GI value food of the present invention. The food or feed can be prepared, for example, by adding the composition of the present invention to a ready-made food or feed, or adding the composition of the present invention previously to the raw material to be used, or blending the composition together during the preparation process. The food of the present invention can also be prepared by adding the composition of the present invention together with the material during cooking of the food (feed, in some cases). The timing or method of adding the composition of the present invention to the food or feed is not particularly limited as long as the food or feed capable of exhibiting the desired effects of the present invention is obtained. '

The content of the composition of the present invention in the food or feed of the present invention is not particularly limited, as long as a given object of the food or feed is accomplished and the desired effects of the present invention are obtained. The weight ratio of the carbohydrates in a food or feed to the composition of the present invention (calculated as the total amount of a *Cyamopsis tetragonolobus* bean protein and a degraded galactomannan) (carbohydrates in the food or feed / composition of the present invention) is preferably from 50/1 to 1/10, more preferably from 40 / 1 to 1/5, and even more preferably from 20 / 1 to 1 / 1, from the viewpoint of obtaining the effects of lowering the GI value of the food or feed.

The effect of the food or feed of lowering a GI value is exhibited by ingesting in a general method for the food or feed, and elevation From the viewpoint of obtaining the effects of lowering the GI value of the food or feed, in blood glucose level is inhibited as compared to the case of the ingestion of the food or feed which does not contain the composition of the present invention. Therefore, the food or feed of the present invention can be effectively used for prevention, amelioration or the like of diabetes of an individual.

The food or feed to which the composition of the present invention is applicable is preferably, but not particularly limited to, a food or feed containing carbohydrates, which has an influence on the GI value. The food or feed includes, for example, rice, bread, Japanese wheat noodle, pasta, garden peas, honey, banana, ice cream, cornflake, orange, yoghurt, Chinese yam and the like. The feed includes all sorts of known feeds such as feeds for livestock, and is preferably used especially for feeds for household pets.

EP 1 639 903 B1

[2] Low-Glycemic Index Food 1 - for reference only -

The present inventors have intensively continued their studies for the purpose of developing a food having a low glycemic index. As a result, the present inventors found that the above-mentioned object and problem can be solved by a food in which the ratio of a polygalactosyl mannose to gliadin is constant, and the ratio of the polygalactosyl mannose to glutenin is constant, or a food in which the ratio of a polygalactose and/or a polygalactose derivative to gliadin is constant, and the ratio of the polygalactose and/or the polygalactose derivative to glutenin is constant.

The polygalactosyl mannose refers to those having a pectinate branching structure in which an α-galactosyl group is bound to the O-6 positions of a β-(1→4) mannan chain of the main chain, and any of those chemically synthesized or derived from a natural product can be utilized. From the viewpoint of saving production cost and use as a food, those derived from natural products are preferable. As a natural product, any raw materials of a plant, an animal, a marine alga and a microbe can be utilized. In view of the availability of the raw material, the plant is preferable. The plant can be exemplified by *Cyamopsis tetragonolobus, Ceratonia siliqua, Gymnocladus dioica, Trigohella foenumgracum, Medicago sativa, Trifolium pratense, Glycine hispida, Actinidia callosa* LINDLEY, *Sesbania bisibinonia* and *Cassia tora* Linn. From the viewpoint of the abundance of the resource and the taste, guar gum and locust bean gum derived from *Cyamopsis tetragonolobus* and *Ceratonia siliqua* are preferable, and guar gum derived from *Cyamopsis tetragonolobus* is most preferable. The molar ratio of the mannose to the galactose (mannose / galactose) in the molecule of the polygalactosyl mannose derived from a plant is preferably from 0.5 to 5.0, more preferably from 1.0 to 3.0, and even more preferably from 1.5 to 2.5, since the food properties such as palatability is good. Moreover, those obtained by hydrolyzing natural mucous substances such as guar gum, locust bean gum, tara gum, cassia gum and sesbania gum which are derived from the above-mentioned plants and industrially utilizable, preferably guar gum, locust bean gum, and sesbania gum, and more preferably guar gum and locust bean gum to make the substance have a low-molecular weight can be used as the polygalactosyl mannose. A method of hydrolysis includes, but not particularly limited to, enzymolysis, acidolysis and the like. Since the degraded product is easily evenly sized in the molecular weight distribution, enzymolysis is preferable. The enzyme used for enzymolysis may be, but not particularly limited to, a commercially available product or a product derived from a natural product, as long as the enzyme is capable of hydrolyzing the mannose straight chain. The enzyme is preferably β-mannanase derived from a bacterium belonging to *Aspergillus, Rhizopus* or the like.

The properties of the polygalactosyl mannose are not particularly limited. The polygalactosyl mannose contains those having a molecular weight distribution of preferably from $1.8 \times 10^3$ to $1.8 \times 10^5$, more preferably from $8.0 \times 10^3$ to $1.0 \times 10^5$, and even more preferably from $1.5 \times 10^4$ to $2.5 \times 10^4$. The polygalactosyl mannose having a molecular weight distribution of $1.8 \times 10^3$ or more is effective in lowering the GI value of a food, and the polygalactosyl mannose having a molecular weight distribution of $1.8 \times 10^5$ or less has a moderate viscosity, thereby giving excellent workability to a food. Here, the molecular weight distribution can be obtained, for example, by a method for determining a molecular weight distribution by high-performance liquid chromatography (column: YMC-Pack Diol-120, YMC Co., Ltd., detector: differential refractometer) using polyethylene glycols (molecular weights: $2.0 \times 10^3$, $2.0 \times 10^4$, and $1.0 \times 10^5$) as molecular weight markers. It is preferable that the polygalactosyl mannose contains a polygalactosyl mannose having the above-mentioned molecular weight distribution in an amount of 70% by weight or more.

Since the polygalactosyl mannose is more effective in lowering a GI value of a food, it is preferable that the polygalactosyl mannose contains a polygalactosyl mannose having a degree of polymerization of from 30 to 40 in an amount of 25% by weight or more. The ratio of the polygalactosyl mannose having a degree of polymerization of from 30 to 40 can be calculated from an average molecular weight obtained by obtaining a molecular weight distribution in the above-mentioned determination method, applying to a calibration curve obtained from the molecular weight markers to numerically express the molecular weights, and averaging the obtained values, and the molecular weights of the mannose and the galactose.

The viscosity of the polygalactosyl mannose is, but not particularly limited to, preferably 50 mPa•s or less, more preferably 30 mPa•s or less, and even more preferably 10 mPa•s or less as determined with a B Type Viscometer in a 0.5(w/v)% aqueous solution at 25°C.

The commercial available product of the polygalactosyl mannose includes SUNFIBER (manufactured by Taiyo Kagaku Co., Ltd.), Fiberon (manufactured by DAINIPPON PHARMACEUTICAL CO., LTD.), Guar Fiber (manufactured by MEIJI SEIKA KAISHA, LTD.), G-Fiber (manufactured by Glico Foods Co., Ltd.) and the like.

The polygalactose of the polygalactose and/or the polygalactose derivative refers to an oligosaccharide or a polysaccharide composed only of galactose. The binding mode is, but not particularly limited to, preferably a polygalactose having a β-(1→3) bond, and more preferably a β-(1→3) bound straight-chain polygalactose.

The polygalactose derivative of the polygalactose and/or the polygalactose derivative refers to, but not particularly limited to, for example, those which are derivatized from a polygalactose. For example, the derivative can be obtained as a natural product from a plant, an animal or the like, in addition to a product derivatized by synthesis. The mode of derivatization includes, but not particularly limited to, modification with a sugar chain comprising glucose,

7

fructose, galactose, arabinose, xylose or the like as a side chain, substitution of a hydroxyl group in the carbohydrates with a sulfonyl group, an amino group, a carboxyl group or the like, and modification to a hydroxyl group in the carbohydrate by means of esterification, acetylation or the like. The polygalactose derivative is preferably a polygalactose having arabinose and/or galactose on the side chain, and more preferably a β-(1→3) bound straight-chain polygalactose having arabinose and/or galactose on the side chain.

The polygalactose and/or the polygalactose derivative is not particularly limited by its origin, and can be originated from a natural product, a synthesized product or the like. For example, the polygalactose derivative is preferably a polygalactose derivative derived from a plant belonging to *Larix,* more preferably a polygalactose derivative derived from *Larix leptolepis, Larix kaempferi, Larix cajanderi, Larix decidu, Larix gmenlinii, Larix griffithiana, Larix sibrica, Larix decudua* or *Larix olgensis,* and even more preferably a polygalactose derivative derived from *Larix leptolepis.*

The viscosity of the polygalactose and/or the polygalactose derivative is preferably from 5 to 15 mPa•s, more preferably from 7 to 14 mPa•s, and even more preferably from 9 to 13 mPa•s as determined with a B Type Viscometer in a 30(w/v)% aqueous solution at 25°C using a rotor No. 1 under the conditions of a rotational speed of 20 rpm. Those having the viscosity within the above range give excellent workability to a food. Here, the viscosity in the case of "and" in "the polygalactose and/or the polygalactose derivative" refers to the viscosity of the aqueous solution of the polygalactose and the polygalactose derivative.

As the polygalactose and/or polygalactose derivative, those having a pH of a 1(w/v)% aqueous solution of from 5 to 7 are preferable. Those having the pH within the above range give excellent workability to a food. Here, the meaning of "and" in "the polygalactose and/or the polygalactose derivative" is similar to the meaning in the above-mentioned viscosity.

The average molecular weight of the polygalactose and/or the polygalactose derivative is preferably, but not particularly limited to, 10000 or more and 120000 or less. The average molecular weight is more preferably 12000 or more and 100000 or less, and even more preferably it is 15000 or more and 25000 or less. Those having an average molecular weight of 10000 or more are effective in lowering a GI value of a food. On the other hand, those having an average molecular weight of 120000 or less have moderate viscosity and giving excellent workability to a food. Here, the average molecular weight is calculated by gel filtration chromatography using a column for gel filtration (for example, Sephacryl S-300 manufactured by Amarsham Pharmacia Biotech), on the basis of a calibration curve obtained from a standard substance. As the standard substance, for example, dextran of a known molecular weight can be used.

As the polygalactose and/or the polygalactose derivative, for example, a polygalactose and/or a polygalactose derivative having an average molecular weight of 10000 or more and 120000 or less, a viscosity of a 30% (w/v) aqueous solution thereof of from 5 to 15 mPa•s at 25°C, as determined with B Type Viscometer, and a pH of a 1% (w/v) aqueous solution thereof of from 5 to 7 can be especially preferably used.

The galactose content in the total carbohydrates in the polygalactose and/or the polygalactose derivative ranges, but not particularly limited to, preferably from 82 to 90% by moL from the viewpoint of being more effective in lowering a GI value of a food. More preferably, the galactose content in the total carbohydrates ranges from 83 to 88% by moL, and even more preferably ranges from 84 to 86% by moL. Here, the galactose content in the total carbohydrates can be obtained, for example, by isolating a polygalactose and/or a polygalactose derivative in the low-glycemic index food of the present invention and determining the monosaccharide composition by acidolyzing the carbohydrates in accordance with HPAE-PAD method. The HPAE-PAD method is conveniently carried out using a saccharide analysis system DXc-500 manufactured by Dionex Corporation.

The gliadin is a protein soluble in 70(v/v)% ethanol or a dilute acid. The molecular weight distribution of the gliadin is usually about from 10000 to 80000. Glutamine and proline are richly contained as amino acid components.

The gliadin is not particularly limited by its origin, and can be originated from a natural product, a fermented product, a synthesized product and the like. From the viewpoint of its use as a food material, those derived from a natural product are preferable, and those derived from *Triticum aestivum* L. are more preferable because of the abundance of the raw material.

The glutenin refers to a protein that is insoluble in water and a neutral salt solution but soluble in a dilute acid and a dilute alkali.

The glutenin is not particularly limited by its origin, and can be originated from a natural product, a fermented product, a synthesized product and the like. From the viewpoint of its use as a food material, those derived from a natural product are preferable, and those derived from *Triticum aestivum* L. are more preferable because of the abundance of the raw material.

The determination methods of the gliadin content and the glutenin content in the low-GI value food are not particularly limited. For example, a method comprising preliminarily extracting albumin and globulin from flour with a salt solution according to the method of Wieser H et al. (Wieser H, Antes S, Seilmeier W: Cereal Chem. Vol. 75, 644-650 (1998)), thereafter extracting gliadin with an aqueous ethanol solution, subsequently extracting a glutenin subunit, and subjecting to analysis by reversed phase HPLC can be applied.

The weight ratio of the polygalactosyl mannose to the gliadin in the low-GI value food (polygalactosyl mannose :

gliadin) is preferably from 1.0 : 0.5 to 1.0 : 25.0, and more preferably from 1.0 : 0.8 to 1.0 : 12.0. When the weight ratio of the polygalactosyl mannose to the gliadin is within the above-mentioned range, a food having a sufficiently low GI value and excellent palatability can be obtained.

The weight ratio of the polygalactosyl mannose to the glutenin in the low-glycemic index food (polygalactosyl mannose : glutenin) is preferably from 1.0 : 0.2 to 1.0 : 15.0, and more preferably from 1.0 : 0.5 to 1.0 : 13.0. When the weight ratio of the polygalactosyl mannose to the glutenin is within the above-mentioned range, a food having a sufficiently low GI value and excellent palatability can be obtained.

The weight ratio of the polygalactose and/or the polygalactose derivative to the gliadin in the low-glycemic index food (polygalactose and/or polygalactose derivative : gliadin) is preferably from 1.0 : 0.5 to 1.0 : 25.0, and more preferably from 1.0 : 0.8 to 1.0 : 12.0. When the weight ratio of the polygalactose and/or the polygalactose derivative to the gliadin is within the above-mentioned range, a food having a sufficiently low GI value and excellent palatability can be obtained.

The weight ratio of the polygalactose and/or the polygalactose derivative to the glutenin in the low-glycemic index food (polygalactose and/or polygalactose derivative : glutenin) is preferably from 1.0 : 0.2 to 1.0 : 15.0, and more preferably from 1.0 : 0.5 to 1.0 : 13.0. When the weight ratio of the polygalactose and/or the polygalactose derivative to the glutenin is within the above-mentioned range, a food having a sufficiently low GI value and excellent palatability can be obtained.

In a more preferred embodiment, the preferred polygalactose and/or preferred polygalactose derivative mentioned above is used in combination with gliadin and/or glutenin in the above-mentioned ratio.

The low-GI value food can be obtained by blending each of the components mentioned above with any raw material of the food, and preparing the desired food according to known methods (for example, the methods used in the food industry). During the preparation, it is also possible to appropriately form the food into any form depending upon the food.

The content of gliadin and glutenin, and one or more compounds (active principles) selected from the group consisting of polygalactosyl mannose, polygalactose and polygalactose derivative is not particularly limited, as long as a given object of the food is accomplished, and the desired effects can be obtained. From the viewpoint of obtaining a food having a sufficiently low GI value, the weight ratio of the carbohydrates to the active principle is preferably from 50/1 to 1/10, more preferably from 40/1 to 1/5.

As to the ingestion of the low-glycemic index food, the ingesting method may be, but not particularly limited to, utilization of the low-glycemic index food alone or concomitant use with other components of a diet.

The low-GI value food can be used in place of any corresponding foods which have been conventionally ingested. The amount of the low-glycemic index food ingested is, for example, preferably from 60 to 210 g, more preferably from 90 to 180 g, and even more preferably from 100 to 150 g for human per day.

[3] Low-Glycemic Index Food 2 - for reference only -

The present inventors have intensively continued their studies for the purpose of developing a food having a low glycemic index. As a result, the present inventors found that the above-mentioned object and problems can be solved by a food in which a polygalactosyl mannose to amylose is in a given ratio, and the polygalactosyl mannose to amylopectin is in a given ratio, or a food in which a polygalactose and/or a polygalactose derivative to amylose is in a given ratio, and the polygalactose and/or the polygalactose derivative to amylopectin is in a given ratio.

The polygalactosyl mannose, the polygalactose and the polygalactose derivative used in this aspect are basically the same as those described in the above [2]. As the polygalactose and/or the polygalactose derivative, the preferred polygalactose and/or polygalactose derivative mentioned above is especially used.

The amylose refers to a straight-chain glucose polymer having one each of a non-reducing terminal and a reducing terminal, wherein glucose is linked via an $\alpha$-(1→4) glucosidic bond, and the molecular weight is usually within a range of from 500000 to 2000000. The amylose also forms a water-insoluble complex with butanol, amyl alcohol, thymol or the like. The amylose has a property of exhibiting blue color (maximum wave length: 650 nm) by iodo-starch reaction.

The amylose is not particularly limited by its origin, and can be a product derived from a natural product, a fermented product, a synthesized product or the like. From the viewpoint of its use as a food material, the product derived from a natural product, especially a product derived from a plant is preferable. Especially products derived from *Zea mays* L., *Solanum tuberosum* L., *Ipomoea batatas* Poiret, *Triticum aestivum* L., *Oryzae sativa* L., *Manihot utilissima* Pohl, Sago palm, *Pueraria hirsuta* Matsum or *Erythronium jeponicum* are more preferable. From the viewpoint of being industrially available in a large amount, those derived from *Zea mays* L., *Solanum tuberosum* L., *Triticum aestivum* L. or *Oryzae sativa* L. are even more preferable.

The determination method of the amylose content in the low-GI value food is not limited, but preferably the content can be determined in accordance with the method of Gibson TS et al. (Gibson TS, Salah VA, McCleary BV: J.

Cereal Sci., Vol. 25, 111-119 (1997)) or the method of Iwata et al. (H Iwata, A Isogai, H Utsunomiya, T Itani, N Nishio: Nippon Nougeikagaku Kaishi, Vol. 77, 1130-1136 (2003)) described below.

Specifically, 1 mL of dimethyl sulfoxide (DMSO) is added to 20 mg of the sample to gelatinize, and the starch thereof is reprecipitated with 6 mL of 95(v/v)% ethanol. The mixture is centrifuged (2000 rpm, 5 minutes). One milliliter of DMSO is added to the precipitates to gelatinize, and 180 mM acetic acid buffer (pH 6.4, containing 900 mM sodium chloride) is added thereto, to make up a given volume of 25 mL of the mixture (solution B). Amyloglucosidase / $\alpha$-amylase is added to 0.5 mL of the solution B, to completely degrade the solute to glucose. This glucose is quantified in accordance with a glucose oxidase method, to give a total amount of the starch. Further, 0.5 mL of a concanavalin A solution (4 mg/mL) is added to 1 mL of the solution B to remove the amylopectin by precipitation. The residual amylose is degraded into glucose, to obtain an amylose content in the same manner as in the case of the total amount of starch. According to this method, the total amount of the starch and the amylose content in the sample, and the amylose content (% by weight) in starch can be obtained. Here, a standard sample having an amylose content of 70.0% by weight is added thereto for adjustment.

The amylopectin refers to a glucose polymer in which glucoses are bound via an $\alpha$-(1→4) glucosidic bond, usually having a branching structure via an $\alpha$-(1→6) glucosidic bond in a ratio of 1 branching per about 25 glucose residues. The molecular weight is usually within a range of from 15000000 to 400000000. The amylopectin also has a property of exhibiting magenta color (maximum wave length: 540 nm) by iodo-starch reaction.

The amylopectin is not particularly limited by its origin, and can be a product derived from a natural product, a fermented product, a synthesized product and the like. From the viewpoint of its use as a food material, a product derived from a natural product, and especially a product derived from a plant is preferable. The products derived from *Zea mays* L., *Solanum tuberosum* L., *Ipomoea batatas* Poiret, *Triticum aestivum* L., *Oryzae sativa* L., *Manihot utilissima* Pohl, Sago palm, *Pueraria hirsuta* Matsum or *Erythronium jeponicum* are more preferable. From the viewpoint of being industrially available in a large amount, the products derived from *Zea mays* L., *Solanum tuberosum* L., *Triticum aestivum* L. or *Oryzae sativa* L. are even more preferable.

The method for determination of the amylopectin content in the low-GI value food is not particularly limited. Preferably, the content can be determined in accordance with the method of Gibson TS et al. (Gibson TS, Salah VA, McCleary BV: J. Cereal Sci., Vol. 25, 111-119 (1997)) or the method of Iwata et al. (H Iwata, A Isogai, H Utsunomiya, T Itani, N Nishio: Nippon Nougeikagaku Kaishi, Vol. 77, 1130-1136 (2003)) described below.

Specifically, 1 mL of DMSO is added to 20 mg of the sample to gelatinize, and the starch thereof is reprecipitated with 6 mL of 95(v/v)% ethanol. The mixture is centrifuged (2000 rpm, 5 minutes). One milliliter of DMSO is added to the precipitates to gelatinize, and 180 mM acetic acid buffer (pH 6.4, containing 900 mM sodium chloride) is added, to make up a given volume of 25 mL of the mixture (solution B). Amyloglucosidase / $\alpha$-amylase is added to 0.5 mL of the solution B, to completely degrade the solute to glucose. The glucose is quantified in accordance with glucose oxidase method, to give a total amount of the starch. Further, 0.5 mL of a concanavalin A solution (4 mg/mL) is added to 1 mL of the solution B to remove the amylopectin by precipitation. The residual amylose is degraded to glucose, to obtain an amylose content in the same manner as in the case of the total amount of starch. An amylopectin content is obtained by subtracting the amylose content from the total amount of starch. Here, a standard sample having an amylose content of 70.0% by weight is added thereto, for adjustment.

The weight ratio of the polygalactosyl mannose to the amylose of the low-glycemic index food (polygalactosyl mannose : amylose) is from 1.0 : 0.3 to 1.0 : 4.7, and preferably from 1.0 : 0.8 to 1.0 : 3.9. When the weight ratio of the polygalactosyl mannose to the amylose is within the above-mentioned range, a food having a sufficiently low GI value and excellent palatability can be obtained.

The weight ratio of the polygalactosyl mannose to the amylopectin of the low-glycemic index food (polygalactosyl mannose: amylopectin) is from 1.0 : 7.8 to 1.0 : 25.3, and preferably from 1.0 : 8.9 to 1.0 : 22.1. When the weight ratio of the polygalactosyl mannose to the amylopectin is within the above-mentioned range, a food having a sufficiently low GI value and excellent palatability can be obtained.

The weight ratio of the polygalactose and/or the polygalactose derivative to the amylose of the low-glycemic index food (polygalactose and/or the polygalactose derivative : amylose) is from 1.0 : 0.3 to 1.0 : 4.7, and preferably from 1.0 : 0.8 to 1.0 : 3.9. When the weight ratio of the polygalactose and/or the polygalactose derivative to the amylose is within the above-mentioned range, a food having a sufficiently low GI value and excellent palatability can be obtained.

The weight ratio of the polygalactose and/or the polygalactose derivative to the amylopectin of the low-glycemic index food (polygalactose and/or the polygalactose derivative : amylopectin) is from 1.0 : 7.8 to 1.0 : 25.3, preferably from 1.0 : 8.9 to 1.0 : 22.1. When the weight ratio of the polygalactose and/or the polygalactose derivative to the amylopectin is within the above-mentioned range, a food having a sufficiently low GI value and excellent palatability can be obtained.

The low-GI value food of the present invention can be obtained by blending each of the components mentioned above with any raw materials of the food, and preparing the desired food according to known methods (for example, the methods used in the food industry). Upon preparation, it is also possible to appropriately prepare the food into any form depending upon the foods.

The contents of the polygalactosyl mannose, or the polygalactose and/or the polygalactose derivative, and the amylose and the amylopectin (active principles) are not particularly limited, as long as a desired object of the food is accomplished, and the desired effects can be obtained. From the viewpoint of obtaining a food having a sufficiently low GI value, the weight ratio of the carbohydrates in the food to the active principle (carbohydrates in the food / active principle) is preferably from 50/1 to 1/10, and more preferably from 40/1 to 1/5.

As to the ingestion of the low-glycemic index food, the ingesting method can be, but not particularly limited to, utilization of the low-glycemic index food alone or concomitant use with other components of a diet.

The low-GI value food is used in place of any corresponding food which is conventionally ingested, corresponding thereto. The amount of the low-glycemic index food ingested is, for example, preferably from 90 to 210 g, more preferably from 120 to 180 g, and even more preferably from 140 to 160 g, for human per day.

The low-GI value food can significantly contribute to lower the load on beta-cells of islets of Langerhans of pancreas for insulin secretion resulting from ingestion of foods and the like. Therefore, since the low-GI value food can be efficiently used for prevention of onset of diabetes or amelioration of diabetes, the low-GI value food is preferably used as a food for specified health use or a food for a diabetic patient. The method of ingesting those foods can be pursuant to the conventional methods applied to those foods.

EXAMPLES

The present invention will be explained in detail hereinbelow by means of the examples, without intending to limit the present invention thereto.

Preparation Example 1-1

A 0.1 N hydrochloric acid was added to 900 g of water, to adjust the pH to 4.5. The amount 0.2 g of β-mannanase derived from *Aspergillus* bacteria (manufactured by Novo Nordisk Bioindustry) and 100 g of guar gum powder (manufactured by Taiyo Kagaku Co., Ltd., high-grade product) were added thereto, and the mixture was mixed, to carry out enzymolysis of guar gum at 40° to 45°C over a period of 24 hours. After the reaction, the mixture was heated at 90°C for 15 minutes, to deactivate the enzyme. The mixture was separated by filtration (suction filtration), to remove insoluble substances. The resulting transparent solution was concentrated under a reduced pressure (Yamato evaporator) (solid content: 20% by weight), and thereafter the concentrate was dried with a spray dryer [manufactured by Ohkawara Kakouki Co., Ltd.], to give 65 g of a degraded galactomannan (degraded galactomannan content: 90% by weight) in the form of powder. Here, the degraded galactomannan contained protein in an amount of 1.5% by weight.

The viscosity of an aqueous solution obtained by dissolving the degraded galactomannan in water having a concentration of 0.5(w/v)% when calculated as the amount of the degraded galactomannan was determined with a B Type Viscometer [manufactured by TOKI SANGYO CO., LTD.] at 25°C. As a result, the viscosity was 2 mPa•s. In addition, the aqueous solution was subjected to high-performance liquid chromatography [column manufactured by YMC Co., Ltd.: YMC-Pack Diol-120] using polyethylene glycols (molecular weights: 2000, 20000 and 100000) as molecular weight markers to obtain an average molecular weight. As a result, the average molecular weight was about 20000.

Preparation Example 1-2

A 0.1 N hydrochloric acid was added to 900 g of water, to adjust the pH to 3. The amount 0.15 g of β-mannanase derived from *Aspergillus* bacteria (manufactured by Novo Nordisk Bioindustry) and 100 g of guar gum powder (manufactured by Taiyo Kagaku Co., Ltd., medium-grade product) were added thereto, and the mixture was mixed, to carry out enzymolysis of guar gum at 40° to 45°C over a period of 24 hours. After the reaction, the mixture was heated at 90°C for 15 minutes, to deactivate the enzyme. The mixture was separated by filtration (suction filtration), to remove insoluble substances. The resulting transparent solution was concentrated under a reduced pressure (Yamato evaporator) (solid content: 20% by weight), and thereafter the concentrate was dried with a spray dryer [manufactured by Ohkawara Kakouki Co., Ltd.], to give 68 g of a degraded galactomannan (degraded galactomannan content: 85% by weight) in the form of powder. Here, the degraded galactomannan contained protein in an amount of 4.1% by weight.

The viscosity of the resulting degraded galactomannan was determined in the same manner as in Preparation Example 1-1. As a result, the viscosity was 3 mPa•s. In addition, the average molecular weight was obtained. As a result, the molecular weight was about 25000.

Preparation Example 1-3

A 0.1 N hydrochloric acid was added to 900 g of water, to adjust the pH to 4. The amount 0.25 g of β-mannanase derived from *Aspergillus* bacteria (manufactured by Novo Nordisk Bioindustry) and 100 g of guar gum powder (manufactured by Taiyo Kagaku Co., Ltd., low-grade product) were added thereto, and the mixture was mixed, to carry out enzymolysis of guar gum at 50° to 55°C over a period of 12 hours. After the reaction, the mixture was heated at 90°C for 15 minutes, to deactivate the enzyme. The mixture was separated by filtration (suction filtration), to remove insoluble substances. The resulting transparent solution was concentrated under a reduced pressure (Yamato evaporator) (solid content: 20% by weight), and thereafter the concentrate was dried with a spray dryer [manufactured by Ohkawara Kakouki Co., Ltd.], to give 70 g of a degraded galactomannan (content of degraded galactomannan: 80% by weight) in the form of powder. Here, the degraded galactomannan contained protein in an amount of 6.7% by weight.

The viscosity was determined for the degraded galactomannan obtained in the same manner as in Preparation Example 1-1. As a result, the obtained viscosity was 9 mPa•s. In addition, the average molecular weight was obtained. As a result, the molecular weight was about 15000.

Preparation Example 1-4

In accordance with the description of Examples of the above-mentioned Japanese Patent publication 05-117156 (page 4, line 3 to page 4, line 10), a hydrolyzed guar gum (degraded galactomannan) was prepared. The average molecular weight was determined in accordance with Preparation Example 1-1. As a result, the average molecular weight obtained was 5500. In addition, the viscosity was 8 mPa•s. The hydrolysate contained degraded galactomannan in an amount of 91% by weight, and the protein in an amount of 0.1% by weight.

Preparation Example 1-5

One-hundred grams of *Cyamopsis tetragonolobus* beans were appropriately pulverized in a food processor. The resulting pulverized beans were added to 1000 g of water, and the mixture was mixed. The soluble components were allowed to elute with keeping the mixture at room temperature for about 2 hours while stirring. Next, a dispersion of the pulverized product was subjected to centrifugation, to remove insoluble components as precipitates. Hydrochloric acid was added to the supernatant obtained, to adjust the pH to 4.5, and the protein was allowed to precipitate. The precipitates were further subjected to centrifugation, and the supernatant was removed. Thereafter, washing was carried out by adding water to the remaining precipitates, to once disperse the precipitates, centrifuging the dispersion again and collecting the precipitates. Here, the procedures were repeated 3 times. The precipitates were dispersed in water, and the pH was then adjusted to 7 by adding sodium hydroxide thereto, to dissolve the precipitates by neutralization. Thereafter the resulting solution was spray-dried, to give 34 g of a *Cyamopsis tetragonolobus* bean protein in the form of powder. The protein content was 91% by weight.

Example 1-1

The amount 4.5 g of the degraded galactomannan of Preparation Example 1-4 and 0.5 g of the *Cyamopsis tetragonolobus* bean protein of Preparation Example 1-5 were mixed, to give a composition for lowering a GI value. The weight ratio of the *Cyamopsis tetragonolobus* bean protein to the degraded galactomannan *(Cyamopsis tetragonolobus* bean protein / degraded galactomannan) was 1 / 8.9.

Example 1-2

The composition for lowering a GI value of Example 1-1 was blended in boiled rice, to make an *onigiri* (rice ball). The weight ratio of the carbohydrates in the rice ball to the composition (carbohydrates in the *onigiri* / composition) was 16/1.

Comparative Examples 1-1 and 1-2

The amount 4.5 g of the degraded galactomannan of Preparation Example 1-4 and 0.5 g of a milk protein (manufactured by Omu milk products co., ltd.) or a soy protein (manufactured by FUJI OIL CO., LTD.) were mixed, to give a comparative composition.

Test Example 1-1

The influence of foods on GI values was studied by asking subjects (20 healthy women: average age: 20, average weight: 57 kg) to ingest various test foods simultaneously together with any of the degraded galactomannan of Preparation Examples 1-1 to 1-4, the *Cyamopsis tetragonolobus* bean protein of Preparation Example 1-5 and the composition for lowering a GI value of Example 1-1 as a test substance.

As the test food, boiled rice, bread, Japanese wheat noodle, pasta or orange was used. The amount of the food in one ingestion was set to be 50 g calculated as the amount of carbohydrates. The amount of each test substance in one ingestion was set to be 5 g for the degraded galactomannan of Preparation Examples 1-1 to 1-4, 0.5 g for the *Cyamopsis tetragonolobus* bean protein of Preparation Example 1-5, and 5 g for the composition for lowering a GI value of Example 1-1, respectively. The test substance was dissolved in 200 mL of water, and the solution was ingested simultaneously with each food.

Blood was taken from the subjects every 30 minutes until 2 hours after the ingestion of the test food and the test substance. The blood glucose level was determined with a glucose-test sensor (manufactured by Sanwa Kagaku Kenkyusho K.K.). Each subject was made to take 50 g of glucose, and the blood glucose level was then separately determined previously in the same manner. Blood glucose level curves for both cases were drawn on the bases of the found values. The area below the blood glucose level curve was obtained by calculating the area below the curve until 2 hours after the ingestion, and a GI value was obtained according to the formula of the "GI value of food or feed" mentioned above.

On the day of the test, the subjects took the test skipping breakfast. Each subject ingested one kind of food arbitrarily. The test was carried out on the different days for each of the different kinds of foods.

The average of the GI values for each food in the case where a test substance was simultaneously ingested is shown in Tables 1-1 and 1-2. In each food, the GI value in the case where only the food was ingested without ingestion of the test substance is shown in the table as "without addition" as a comparative control.

Table 1-1

| Test Food | Test Substance | GI Value (average) |
|---|---|---|
| Boiled Rice | Without addition | 88 |
| | Degraded Galactomannan of Preparation Example 1-1 | 49 |
| | Degraded Galactomannan of Preparation Example 1-2 | 47 |
| | Degraded Galactomannan of Preparation Example 1-3 | 43 |
| | Degraded Galactomannan of Preparation Example 1-4 | 73 |
| | *Cyamopsis tetragonolobus* Bean Protein of Preparation Example 1-5 | 85 |
| | Composition for lowering a GI value of Example 1-1 | 40 |
| Bread | Without addition | 95 |
| | Degraded Galactomannan of Preparation Example 1-1 | 58 |
| | Degraded Galactomannan of Preparation Example 1-2 | 59 |
| | Degraded Galactomannan of Preparation Example 1-3 | 49 |
| | Degraded Galactomannan of Preparation Example 1-4 | 72 |
| | *Cyamopsis tetragonolobus* Bean Protein of Preparation Example 1-5 | 91 |
| | Composition for lowering GI value of Example 1-1 | 45 |
| Japanese Wheat Noodle | Without addition | 57 |
| | Degraded Galactomannan of Preparation Example 1-1 | 42 |
| | Degraded Galactomannan of Preparation Example 1-2 | 41 |
| | Degraded Galactomannan of Preparation Example 1-3 | 39 |
| | Degraded Galactomannan of Preparation Example 1-4 | 50 |
| | *Cyamopsis tetragonolobus* Bean Protein of Preparation Example 1-5 | 55 |
| | Composition for Lowering GI value of Example 1-1 | 38 |

Table 1-2

| Test Food | Test Substance | GI Value (average) |
|---|---|---|
| Pasta | Without addition | 65 |
| | Degraded Galactomannan of Preparation Example 1-1 | 47 |

(continued)

| Test Food | Test Substance | GI Value (average) |
|---|---|---|
| | Degraded Galactomannan of Preparation Example 1-2 | 46 |
| | Degraded Galactomannan of Preparation Example 1-3 | 42 |
| | Degraded Galactomannan of Preparation Example 1-4 | 55 |
| | *Cyancopsis tetragonolobus* Bean Protein of Preparation Example 1-5 | 63 |
| | Composition for Lowering GI value of Example 1-1 | 40 |
| Orange | Without addition | 31 |
| | Degraded Galactomannan of Preparation Example 1-1 | 22 |
| | Degraded Galactomannan of Preparation Example 1-2 | 22 |
| | Degraded Galactomannan of Preparation Example 1-3 | 20 |
| | Degraded Galactomannan of Preparation Example 1-4 | 28 |
| | *Cyamopsis tetragonolobus* Bean Protein of Preparation Example 1-5 | 29 |
| | Composition for Lowering GI value of Example 1-1 | 19 |

It can be seen from Tables 1-1 and 1-2 that each of the foods shows lowered GI value when the degraded galactomannan of Preparation Examples 1-1 to 1-4 and the composition for lowering a GI value of Example 1-1 are ingested at the same time with each food as compared to that of without addition, and that the effects of lowering of GI values are even higher especially when the composition for lowering a GI value of Example 1-1 containing both the degraded galactomannan and the *Cyamopsis tetragonolobus* bean protein is ingested. It can also be seen that the effect of lowering of a GI value cannot be accomplished only with the *Cyamopsis tetragonolobus* bean protein of Preparation Example 1-5.

Test Example 1-2

The influences on the GI values of foods were studied in accordance with Test Example 1-1 using any of the composition for lowering a GI value of Example 1-1 and the comparative compositions of Comparative Examples 1-1 and 1-2 as a test substance. The results are shown in Table 1-3.

Table 1-3

| Test Food | Test Substance | GI Value (average) |
|---|---|---|
| Boiled Rice | Without addition | 88 |
| | Comparative Composition of Comparative Example 1-1 (Milk Protein + Degraded Galactomannan) | 73 |
| | Comparative Composition of Comparative Example 1-2 (Soy Protein + Degraded Galactomannan) | 71 |
| | Composition for lowering a GI Value of Example 1-1 (*Cyamopsis tetragonolobus* Bean Protein + Degraded Galactomannan) | 40 |
| Bread | Without addition | 95 |
| | Comparative Composition of Comparative Example 1-1 (Milk Protein + Degraded Galactomannan) | 72 |
| | Comparative Composition of Comparative Example 1-2 (Soy Protein + Degraded Galactomannan) | 70 |
| | Composition for lowering a GI Value of Example 1-1 (*Cyamopsis tetragonolobus* Bean Protein + Degraded Galactomannan) | 45 |

It can be seen from Table 1-3 that in the case of the comparative composition containing a milk protein or a soy protein and the degraded galactomannan of Preparation Example 1-4, the GI value is similar to that in the case where merely the degraded galactomannan and a food are simultaneously ingested (see Table 1-1), and that synergistic effects cannot be especially obtained by concomitant use of those proteins. On the other hand, in the case of the composition for lowering a GI value of Example 1-1, a prominent effect of lowering of GI values is accomplished as compared to that of the case where the degraded galactomannan and a food are simultaneously ingested. It can be seen from the above that the effects of the composition for lowering a GI value of Example 1-1 are owing to the synergistic effects of the degraded galactomannan and the *Cyamopsis tetragonolobus* bean protein.

Preparation Example 2-1 Preparation of a Polygalactose Derivative - for reference -

The amount 2000 kg of *Larix leptolepis* chips was soaked in cold water to carry out extraction for 1 hour, and the extract obtained was filtered, to remove insoluble substances. Subsequently, substances having low molecular weights were removed by ultrafiltration using a filter having a fractionation molecular weight of 10000, and thereafter dehydrated and dried, to give 153 kg of a polygalactose derivative in the form of white powder. The properties of the resulting polygalactose derivative were a viscosity of 11.5 mPa•s (in 30(w/v)%, at 25°C, as determined with B Type Viscometer), a pH of 5.2 (in 1(w/v)%), and a dietary fiber content of 94.2% by weight. The resulting polygalactose derivative had a molecular weight distribution of from 12000 to 100000, an average molecular weight of 20000, and a galactose content in the total carbohydrates of 86.1 % by moL. Furthermore, the structure was a straight-chain polygalactose in which a side chain has galactose and arabinose thereon, and the main chain binds via β-(1→3) bond.
Here, the viscosity of the resulting polygalactose derivative was determined with a B Type Viscometer using a rotor No. 1, under the conditions of 20 rpm. The pH was determined with a pH meter, and the dietary fiber content was determined in accordance with AOAC method.
The molecular weight distribution and the average molecular weight were evaluated using size exclusion gel filtration method. Specifically, 10 mg of a polygalactose derivative was dissolved in 5.0 mL of a 0.1 N aqueous NaCL solution, and the solution was poured into a Sephacryl S-300 column (2.3 cm × 110 cm) equilibrated with a 0.1 N aqueous NaCL solution. Elution was carried out at a flow rate of 0.5 mL/minute. The eluate was collected in an amount of 7.0 mL each, and the sugar in each fraction was separately detected by phenol-sulfuric acid method. The gel filtration column was examined using standard dextran of a known molecular weight, and an average molecular weight was obtained from a semi-logarithmic graph.
The monosaccharide composition was assayed after acidolysis by HPAE-PAD method using a saccharide analysis system DXc-500 manufactured by Dionex Corporation. The detailed results were galactose 86.1% by moL, arabinose 12.2% by moL, glucose 0.2% by moL, and other carbohydrates 1.5% by moL. Conformational analysis of the polygalactose derivative was carried out by methylation analysis and NMR analysis.

Example 2-1 - for reference -

The amount 180 kg of gliadin (trade name: GLIADIN, manufactured by ASAMA CHEMICAL CO., LTD., derived from *Triticum aestivum* L.), 100 kg of glutenin (trade name: GLUTENIN, manufactured by ASAMA CHEMICAL CO., LTD., derived from *Triticum aestivum* L.) and 10 kg of polygalactosyl mannose (trade name: SUNFIBER, containing a polygalactosyl mannose having a molecular weight distribution of $1.8 \times 10^3$ in an amount of 80% by weight or more, manufactured by Taiyo Kagaku Co., Ltd., derived from *Cyamopsis tetragonolobus*) were mixed with a Nauta Mixer (manufactured by HOSOKAWA Micron Corporation) for 30 minutes, to give 288.0 kg of a low-glycemic index food (inventive product 2-1). Here, the product 2-1 had a weight ratio of the polygalactosyl mannose to the gliadin of 1.0 : 18.0, and a weight ratio of the polygalactosyl mannose to the glutenin of 1.0 : 10.0.

Example 2-2 - for reference -

The amount 465 g of water was added to 642 g of strong flour (manufactured by Nisshin Flour Milling Inc., gliadin content: 3.9% by weight, glutenin content: 1.6% by weight), 35 g of sugar, 13 g of skim milk, 11 g of table salt, 33 g of unsalted butter, 10 g of bakers' yeast and 30 g of polygalactosyl mannose (trade name: SUNFIBER, manufactured by Taiyo Kagaku Co., Ltd., derived from *Cyamopsis tetragonolobus*), and the mixture was subjected to breadmaking with an automated breadmaker (manufactured by ZOJIRUSHI CORPORATION), to give 1100.5 g of a low-glycemic index food (bread) (inventive product 2-2). Here, the product 2-2 had a weight ratio of the polygalactosyl mannose to the gliadin of 1.0 : 0.8, and a weight ratio of the polygalactosyl mannose to the glutenin of 1.0 : 0.3.

Example 2-3 - for reference -

The amount 180 kg of gliadin (trade name: GLIADIN, manufactured by ASAMA CHEMICAL CO., LTD., derived from *Triticum aestivum* L.), 100 kg of glutenin (trade name: GLUTENIN, manufactured by ASAMA CHEMICAL CO., LTD., derived from *Triticum aestivum* L.) and 10 kg of the polygalactose derivative obtained in Preparation Example 2-1 were mixed with a Nauta Mixer (manufactured by HOSOKAWA Micron Corporation) for 30 minutes, to give 288.3 kg of a low-glycemic index food (inventive product 2-3). Here, the product 2-3 had a weight ratio of the polygalactose derivative to the gliadin of 1.0 : 18.0, and a weight ratio of the polygalactose derivative to the glutenin of 1.0 : 10.0.

Example 2-4 - for reference -

The amount 465 g of water was added to 642 g of strong flour (manufactured by Nisshin Flour Milling Inc., gliadin content: 3.9% by weight, glutenin content: 1.6% by weight), 35 g of sugar, 13 g of skim milk, 11 g of table salt, 33 g of unsalted butter, 10 g of bakers' yeast and 30 g of the polygalactose derivative obtained in Preparation Example 2-1, and the mixture was subjected to breadmaking with an automated breadmaker (manufactured by ZOJIRUSHI CORPORATION), to give 1100.5 g of a low-glycemic index food (bread) (inventive product 2-4). Here, the product 2-4 had a weight ratio of the polygalactosyl mannose to the gliadin of 1.0 : 0.8, and a weight ratio of the polygalactosyl mannose to the glutenin of 1.0 : 0.3.

Example 2-5 - for reference -

The amount 30 g of polygalactosyl mannose (trade name: SUNFIBER, manufactured by Taiyo Kagaku Co., Ltd., derived from *Cyamopsis tetragonolobus*), 10 g of *kansui* powder, 10 g of table salt, 330 g of water, and 20 g of 99 (v/v)% ethanol were mixed with 1000 g of mellower strong flour (manufactured by Nisshin Flour Milling Inc., gliadin content: 3.4% by weight, glutenin content: 1.4% by weight), and the mixture was kneaded with a mixer for 15 minutes, the kneaded mixture was rolled, and cut (final thickness of a noodle band: 1.4 mm, cutter # 20 edge) according to a conventional method to give Chinese noodles. The amount 120 g of resulting Chinese noodles were tightly sealed in a plastic bag, and the noodles were aged at 20°C for 24 hours, to give raw Chinese noodles (low-glycemic index food). The raw Chinese noodles had a weight ratio of the polygalactosyl mannose to the gliadin of 1 : 1.1, and a weight ratio of the polygalactosyl mannose to the glutenin of 1 : 0.5.

Example 2-6 - for reference -

Thirty grams of polygalactosyl mannose (trade name: SUNFIBER, manufactured by Taiyo Kagaku Co., Ltd., derived from *Cyamopsis tetragonolobus*) and 300 g of water were added to 1000 g of durum wheat flour (manufactured by Nisshin Flour Milling Inc., gliadin content: 4.9% by weight, glutenin content: 2.8% by weight), and dry noodles of spaghetti (low-glycemic index food) having a water content of 13% by weight was prepared from the mixture according to a conventional method. The dry noodles had a weight ratio of the polygalactosyl mannose to the gliadin of 1 : 1.6, and a weight ratio of the polygalactosyl mannose to the glutenin of 1 : 0.9.

Comparative Example 2-1 - for reference -

The amount 120 kg of gliadin (trade name: GLIADIN, manufactured by ASAMA CHEMICAL CO., LTD., derived from *Triticum aestivum* L.), 80 kg of glutenin (trade name: GLUTENIN, manufactured by ASAMA CHEMICAL CO., LTD., derived from *Triticum aestivum* L.) and 4 kg of polygalactosyl mannose (trade name: SUNFIBER, manufactured by Taiyo Kagaku Co., Ltd., derived from *Cyamopsis tetragonolobus*) were mixed with a Nauta Mixer (manufactured by HOSOKAWA Micron Corporation) for 30 minutes, to give 202.9 kg of a low-glycemic index food (comparative product 2-1). Here, the comparative product 2-1 had a weight ratio of the polygalactosyl mannose to the gliadin of 1.0 : 30.0, and a weight ratio of the polygalactosyl mannose to the glutenin of 1.0 : 20.0.

Comparative Example 2-2 - for reference -

The amount 465 g of water was added to 642 g of strong flour (manufactured by Nisshin Flour Milling Inc., gliadin content: 3.9% by weight, glutenin content: 1.6% by weight), 35 g of sugar, 13 g of skim milk, 11 g of table salt, 33 g of unsalted butter, 10 g of bakers' yeast and 0.5 g of polygalactosyl mannose (trade name: SUNFIBER, manufactured by Taiyo Kagaku Co., Ltd., derived from *Cyamopsis tetragonolobus*), and the mixture was subjected to breadmaking with an automated breadmaker (manufactured by ZOJIRUSHI CORPORATION), to give 1088.5 g of a low-glycemic index food (bread) (comparative product 2-2). Here, the comparative product 2-2 had a weight ratio of the polygalactosyl mannose to the gliadin of the comparative product 2-2 of 1.0 : 49.9, and a weight ratio of the polygalactosyl mannose to the glutenin of 1.0 : 20.4.

Comparative Example 2-3 - for reference -

The amount 120 kg of gliadin (trade name: GLIADIN, manufactured by ASAMA CHEMICAL CO., LTD., derived from *Triticum aestivum* L.), 80 kg of glutenin (trade name: GLUTENIN, manufactured by ASAMA CHEMICAL CO., LTD., derived from *Triticum aestivum* L.) and 4 kg of the polygalactose derivative obtained in Preparation Example 2-1 were mixed with a Nauta Mixer (manufactured by HOSOKAWA Micron Corporation) for 30 minutes, to give 203.1 kg of a low-glycemic index food of the present invention (comparative product 2-3). Here, the comparative product 2-3 had a weight ratio of the polygalactose derivative to the gliadin of 1.0 : 30.0, and a weight ratio of the polygalactose derivative to the glutenin of 1.0 : 20.0.

Comparative Example 2-4 - for reference -

The amount 465 g of water was added to 642 g of strong flour (manufactured by Nisshin Flour Milling Inc., gliadin content: 3.9% by weight, glutenin content: 1.6% by weight), 35 g of sugar, 13 g of skim milk, 11 g of table salt, 33 g of unsalted butter, 10 g of bakers' yeast and 0.5 g of the polygalactose derivative obtained in Preparation Example 2-1, and the mixture was subjected to breadmaking with an automated breadmaker (manufactured by ZOJIRUSHI CORPORATION), to give 1089.7 g of a low-glycemic index food (bread) (comparative product 2-4). Here, the comparative product 2-4 had a weight ratio of the polygalactosyl mannose to the gliadin of 1.0 : 49.9, and the weight ratio of the polygalactosyl mannose to the glutenin of 1.0 : 20.4.

Test Example 2-1 - for reference -

With 14 normal healthy subjects without smoking habit (average age: 46.7, men: 7 members, women: 7 members, average BMI: 25.2 kg/m$^2$, average fasting blood glucose: 5.3 mmoL/L), clinical trial was carried out for the effects on the postprandial glycemic index. The trial was carried out according to randomized cross-over design in which the subjects were asked to ingest a standard food, a control food or a test food. As the standard food, a single glucose solution containing 50 g of glucose (manufactured by Medic Diagnostic Laboratory, trade name: Medic Orange 50 Glucose Tolerance Test Beverage, glucose content: 50 g/each) was ingested as standard carbohydrates. As the control food, bread reported by Wolever et al. was ingested so as to contain carbohydrates in an amount of 50 g per serving (see, for example, the above-mentioned K. Doi, et al. Eds., *Shokumotsu Sen-i* (*Dietary Fiber*)). As the test food, the product 2-1, the product 2-2, the product 2-3, the product 2-4, the comparative product 2-1, the comparative product 2-2, the comparative product 2-3, or the comparative product 2-4 was ingested so as to contain carbohydrates in an amount of 50 g per serving. Here, 63 g of the product 2-1, the product 2-3, the comparative product 2-1 and the comparative product 2-3 correspond to 50 g of carbohydrates, and 203 g per serving of the product 2-2, the product 2-4, the comparative product 2-2 and the comparative product 2-4 correspond to 50 g of the amount of ingestion of carbohydrates.

The subjects were asked to fast, starting from 12 hours before the day of the trial, and thereafter blood samples were taken. After taking the blood samples, the subjects were asked to ingest the control food or the test food, and blood samples were taken every 15 minutes until 2 hours after eating. The blood glucose level of the blood sample taken was determined, and the blood glucose level was recorded with passage of time.

The area below the curve of blood glucose level until 2 hours after eating in the case where the standard food, the control food and the test food were ingested was obtained, to calculate the glycemic index on the basis of the standard food. Here, the glycemic index of the standard food was defined as 100. The average of the glycemic index of each subject is shown in Table 2-1 for each food.

Table 2-1

| Ingested Food | Glycemic Index |
|---|---|
| Control | 94.3 |
| Product 2-1 | 59.2 |
| Product 2-2 | 67.8 |
| Product 2-3 | 58.8 |
| Product 2-4 | 65.2 |
| Comparative Product 2-1 | 90.3 |
| Comparative Product 2-2 | 87.9 |
| Comparative Product 2-3 | 91.4 |
| Comparative Product 2-4 | 88.8 |

As shown in Table 2-1, the products 2-1 to 2-4 showed low glycemic indexes of 70 or less as compared to those of the comparative products 2-1 to 2-4 and the control food.

Test Example 2-2 - for reference -

Clinical trial was carried out on the effect on the postprandial glycemic indexes with 16 patients with insulin-dependent diabetes mellitus who neither have smoking habit nor are subjected to medication (average age: 48.9, men: 8 members, women: 8 members, average BMI: 26.6 kg/ $m^2$, average fasting blood glucose: 9.3 mmoL/L). The trial was carried out according to randomized cross-over design in which the subjects were asked to ingest a control food or a test food. As the control food, bread reported by Wolever et al. was ingested so as to contain carbohydrates in an amount of 50 g per serving. As the test food, the products 2-1 to 2-8 (the product 1, the product 2, the product 3, the product 4, the comparative product 1, the comparative product 2, the comparative product 3 or the comparative product 4) were ingested so as to contain carbohydrates in an amount of 50 g per serving. Here, 63 g of the product 2-1, the product 2-3, the comparative product 2-1 and the comparative product 2-3 correspond to 50 g of carbohydrates, and 203 g per serving of the product 2-2, the product 2-4, the comparative product 2-2 and the comparative product 2-4 correspond to 50 g of the amount of ingestion of carbohydrates.

The subjects were asked to fast, starting from 12 hours before the day of trial, and thereafter blood samples were taken. After taking the blood samples, the subjects were asked to ingest the test food, and blood samples were taken every 30 minutes until 3 hours after eating. The blood glucose level of the blood sample taken was determined, and the blood glucose level was recorded with the passage of time.

The area below the curve of blood glucose level until 2 hours and 3 hours after eating in the case where the control food and the test food were ingested was obtained, to calculate the glycemic index on the basis of the control food. Here, the glycemic index of the control food was defined as 100. The results are shown in Table 2-2.

Table 2-2

| Ingested Food | Glycemic Index | |
| --- | --- | --- |
| | After 2 hours | After 3 hours |
| Control | 100.0 | 100.0 |
| Product 2-1 | 57.7 | 59.7 |
| Product 2-2 | 57.5 | 59.5 |
| Product 2-3 | 58.8 | 59.8 |
| Product 2-4 | 58.5 | 59.5 |
| Comparative Product 2-1 | 89.3 | 90.3 |
| Comparative Product 2-2 | 90.4 | 91.4 |
| Comparative Product 2-3 | 91.5 | 92.5 |
| Comparative Product 2-4 | 88.9 | 89.9 |

As shown in Table 2-2, the products 2-1 to 2-4 showed low glycemic indexes of 60 or less as compared to those of the comparative products 2-1 to 2-4 and the control food.

It can be seen from Test Examples 2-1 and 2-2 that the products are low-glycemic index foods which show low glycemic indexes.

Example 3-1 - for reference -

Ten kilograms of amylose (trade name: amylose A, manufactured by Nakalai Tesque, Inc., derived from *Zea mays* L.), 190 kg of amylopectin (trade name: amylopectin, manufactured by Nakalai Tesque, Inc., derived from *Solanum tuberosum* L.) and 10 kg of polygalactosyl mannose (trade name: SUNFIBER, manufactured by Taiyo Kagaku Co., Ltd., derived from *Cyamopsis tetragonolobus*) were mixed with a Nauta Mixer (manufactured by HOSOKAWA Micron Corporation) for 30 minutes, to give 209.0 kg of a low-glycemic index food (inventive product 3-1). Here, the product 3-1 had a weight ratio of the polygalactosyl mannose to the amylose of 1.0 : 1.0, and a weight ratio of the polygalactosyl mannose to the amylopectin of 1.0 : 19.0.

Example 3-2 - for reference -

The amount 1200 g of water was added to 800 g of polished rice (trade name: Mie Koshihikari, manufactured by Matsusaka Beikoku, amylose content: 15.1% by weight, amylopectin content: 60.2% by weight) and 30 g of polygalactosyl mannose (trade name: SUNFIBER, manufactured by Taiyo Kagaku Co., Ltd., derived from *Cyamopsis tetragonolobus*), and the mixture was cooked with an electric rice cooker (manufactured by SANYO Electric Co., Ltd.),

to give 1928.5 g of a low-glycemic index food (boiled rice) of the present invention (inventive product 3-2). Here, the product 3-2 had a weight ratio of the polygalactosyl mannose to the amylose of 1.0 : 3.3, and a weight ratio of the polygalactosyl mannose to the amylopectin of 1.0 : 13.0.

Example 3-3 - for reference -

The amount 10 kg of amylose (trade name: amylose A, manufactured by Nakalai Tesque, Inc., derived from *Zea mays* L.), 190 kg of amylopectin (trade name: amylopectin, manufactured by Nakalai Tesque, Inc., derived from *Solanum tuberosum* L.) and 10 kg of the polygalactose derivative obtained in Preparation Example 2-1 were mixed with a Nauta Mixer (manufactured by HOSOKAWA Micron Corporation) for 30 minutes, to give 208.8 kg of a low-glycemic index food (inventive product 3-3). Here, the product 3-3 had a weight ratio of the polygalactose derivative to the amylose of 1.0 : 1.1, and a weight ratio of the polygalactose derivative to the amylopectin of 1.0 : 19.4.

Example 3-4 - for reference -

The amount 1200 g of water was added to 800 g of polished rice (trade name: Mie Koshihikari, manufactured by Matsusaka Beikoku, amylose content: 15.1% by weight, amylopectin content: 60.2% by weight) and 30 g of the polygalactose derivative obtained in Preparation Example 2-1, and the mixture was cooked with an electric rice cooker (manufactured by SANYO Electric Co., Ltd.), to give 1927.3 g of a low-glycemic index food (boiled rice) of the present invention (inventive product 3-4). Here, the product 3-4 had a weight ratio of the polygalactose derivative to the amylose of the product 3-4 of 1.0 : 3.2, and a weight ratio of the polygalactose derivative to the amylopectin of 1.0 : 13.4.

Comparative Example 3-1 - for reference -

The amount 20 kg of amylose (trade name: amylose A, manufactured by Nakalai Tesque, Inc., derived from *Zea mays* L.), 106 kg of amylopectin (trade name: amylopectin, manufactured by Nakalai Tesque, Inc., derived from *Solanum tuberosum* L.) and 4 kg of polygalactosyl mannose (trade name: SUNFIBER, manufactured by Taiyo Kagaku Co., Ltd., derived from *Cyamopsis tetragonolobus*) were mixed with a Nauta Mixer (manufactured by HOSOKAWA Micron Corporation) for 30 minutes, to give 126.5 kg of a comparative food (comparative product 3-1). Here, the comparative product 3-1 had a weight ratio of the polygalactosyl mannose to the amylose of 1.0 : 5.0, and a weight ratio of the polygalactosyl mannose to the amylopectin of 1.0 : 26.5.

Comparative Example 3-2 - for reference -

The amount 1200 g of water was added to 800 g of polished rice (trade name: Mie Koshihikari, manufactured by Matsusaka Beikoku, amylose content: 15.1% by weight, amylopectin content: 60.2% by weight) and 15 g of polygalactosyl mannose (trade name: SUNFIBER, manufactured by Taiyo Kagaku Co., Ltd., derived from *Cyamopsis tetragonolobus*)*,* and the mixture was cooked with an electric rice cooker (manufactured by SANYO Electric Co., Ltd.), to give 1914.3 g of a comparative food (comparative product 3-2). Here, the comparative product 3-2 had a weight ratio of the polygalactosyl mannose to the amylose of 1.0 : 6.5, and a weight ratio of the polygalactosyl mannose to the amylopectin of 1.0 : 26.0.

Comparative Example 3-3 - for reference -

The amount 20 kg of amylose (trade name: amylose A, manufactured by Nakalai Tesque, Inc., derived from *Zea mays* L.), 106 kg of amylopectin (trade name: amylopectin, manufactured by Nakalai Tesque, Inc., derived from *Solanum tuberosum* L.) and 4 kg of the polygalactose derivative obtained in Preparation Example 2-1 were mixed with a Nauta Mixer (manufactured by HOSOKAWA Micron Corporation) for 30 minutes, to give 125.6 kg of a comparative food (comparative product 3-3). Here, the comparative product 3-3 had a weight ratio of the polygalactose derivative to the amylose of 1.0: 5.3, and a weight ratio of the polygalactose derivative to the amylopectin of 1.0: 26.4.

Comparative Example 3-4 - for reference -

The amount 1200 g of water was added to 800 g of polished rice (trade name: Mie Koshihikari, manufactured by Matsusaka Beikoku, amylose content: 15.1% by weight, amylopectin content: 60.2% by weight) and 15 g of the polygalactose derivative obtained in Preparation Example 2-1, and the mixture was cooked with an electric rice cooker (manufactured by SANYO Electric Co., Ltd.), to give 1910.2 g of a comparative food (boiled rice) (comparative product 3-4). Here, the comparative product 3-4 had a weight ratio of the polygalactose derivative to the amylose of the comparative product 3-4 of 1.0 : 6.7, and a weight ratio of the polygalactose derivative to the amylopectin of 1.0 : 26.3.

Test Example 3-1 - for reference -

Clinical trial was carried out for the effect on postprandial glycemic indexes with 12 normal healthy subjects who do not have smoking habit (average age: 46.5, men: 6 members, women: 6 members, average BMI: 25.0 kg/m$^2$, average fasting blood glucose: 5.2 mmoL/L). The test was carried out according to randomized cross-over design in which the subjects were asked to ingest a standard food, a control food or a test food. As the standard food, a single glucose solution containing 50 g of glucose (manufactured by Medic Diagnostic Laboratory, trade name: Medic Orange 50 Glucose Tolerance Test Beverage, glucose content: 50 g/each) was ingested as standard carbohydrates. As the control food, bread reported by Wolever et al. was ingested so as to contain carbohydrates in an amount of 50 g per serving (see, for example, above-mentioned K. Doi, et al. Eds., *Shokumotsu Sen-i* (*Dietary Fiber*)). As the test food, the product 3-1, the product 3-2, the product 3-3, the product 3-4, the comparative product 3-1, the comparative product 3-2, the comparative product 3-3, or the comparative product 3-4 was ingested so as to contain carbohydrates in an amount of 50 g per serving. Here, 63 g of the product 3-1, the product 3-3, the comparative product 3-1 and the comparative product 3-3 correspond to 50 g of carbohydrates, and 203 g per serving of the product 3-2, the product 3-4, the comparative product 3-2 and the comparative product 3-4 correspond to 50 g of carbohydrates.

The subjects were asked to fast, starting from 12 hours before the day of trial, and thereafter blood samples were taken. After taking the blood samples, the subjects were asked to ingest the test food, and blood samples were taken every 15 minutes until 2 hours after eating. The blood glucose level of the blood sample taken was determined, and the blood glucose level was recorded with passage of time.

The area below the curve of blood glucose level until 2 hours after eating in the case where the standard food, the control food and the test food were ingested was obtained, to calculate the glycemic index on the basis of the standard food. Here, the glycemic index of the standard food was defined as 100. The results are shown in Table 3-1.

Table 3-1

| Ingested Food | Glycemic Index |
|---|---|
| Control | 93.3 |
| Product 3-1 | 59.3 |
| Product 3-2 . | 67.5 |
| Product 3-3 | 58.9 |
| Product 3-4 | 65.4 |
| Comparative Product 3-1 | 90.1 |
| Comparative Product 3-2 | 87.5 |
| Comparative Product 3-3 | 91.3 |
| Comparative Product 3-4 | 88.6 |

As shown in Table 3-1, the products 3-1 to 3-4 showed low glycemic indexes of 70 or less as compared to those of the comparative products 3-1 to 3-4 and the control food.

Test Example 3-2 - for reference -

Clinical trial was carried out for the effect on postprandial glycemic indexes with 10 patients with insulin-dependent diabetes mellitus who neither have smoking habits nor are subjected to medication (average age: 48.3, men: 5 members, women: 5 members, average BMI: 26.9 kg/ m$^2$, average fasting blood glucose: 9.1 mmoL/L). The test was carried out according to randomized cross-over design in which the subjects were asked to ingest a control food or a test food. As the control food, bread reported by Wolever et al. was ingested so as to contain carbohydrates in an amount of 50 g per serving. As the test food, the product 3-1, the product 3-2, the product 3-3, the product 3-4, the comparative product 3-1, the comparative product 3-2, the comparative product 3-3 and the comparative product 3-4 were ingested so as to contain carbohydrates in an amount of 50 g per serving. Here, 63 g of the product 3-1, the product 3-3, the comparative product 3-1 and the comparative product 3-3 correspond to 50 g of carbohydrates, and 203 g per serving of the product 3-2, the product 3-4, the comparative product 3-2 and the comparative product 3-4 correspond to 50 g of carbohydrates.

The subjects were asked to fast, starting from 12 hours before the day of trial, and thereafter blood samples were taken. After taking the blood samples, the subjects were asked to ingest the control food or the test food, and blood samples were taken every 30 minutes until 3 hours after eating. The blood glucose level of the blood sample taken was determined, and the blood glucose level was recorded with passage of time.

The area below the curve of blood glucose level until 3 hours after eating in the case where the control food and the test food were ingested was obtained, to calculate the glycemic index on the basis of the control food. Here, the glycemic index of the control food was defined as 100. The results are shown in Table 3-2.

Table 3-2

| Ingested Food | Glycemic Index |
| --- | --- |
| Control | 100 |
| Product 3-1 | 59.9 |
| Product 3-2 | 59.8 |
| Product 3-3 | 59.7 |
| Product 3-4 | 59.6 |
| Comparative Product 3-1 | 90.1 |
| Comparative Product 3-2 | 91.3 |
| Comparative Product 3-3 | 92.4 |
| Comparative Product 3-4 | 89.6 |

As shown in Table 3-2, the products 3-1 to 3-4 show low glycemic indexes of 60 or less as compared to those of the comparative products 3-1 to 3-4 and the control food.

INDUSTRIAL APPLICABILITY

According to the present invention, a composition for lowering a glycemic index of a food or feed which is simply applicable, effective and safe for foods or feed, and a low-glycemic index food and feed are provided. Those compositions and the like are effective in prevention of onset of diabetes or amelioration of diabetes of a living body, and significantly contribute to the food industry or the medical industry.

**Claims**

1. A composition for lowering a glycemic index (GI value) of a food or feed, comprising a *Cyamopsis tetragonolobus* bean protein and a hydrolysed galactomannan.

2. The composition according to claim 1, wherein the weight ratio of the *Cyamopsis tetragonolobus* bean protein to the hydrolysed galactomannan (*Cyamopsis tetragonolobus* bean protein / hydrolysed galactomannan) is from 1/99 to 1/5.

3. The composition according to claim 1 or 2, wherein the average molecular weight of the hydrolysed galactomannan is from 2000 to 100000.

4. The composition according to any one of claims 1 to 3 wherein the viscosity of a 0.5 (w/v)% aqueous solution of the hydrolysed galactomamnan is 50 mPa·s or less at 25°C when determined with a B Type Viscometer.

5. A food or feed comprising the composition as defined in any one of claims 1 to 4.

6. Use of the food as defined in claim 5 for the manufacture of a food for specified health use or a food for a diabetic patient.

**Patentansprüche**

1. Zusammensetzung zur Senkung eines glykämischen Indexes (GI-Wert) eines Nahrungs- oder Futtermittels, die ein Protein der Bohne Cyamopsis tetragonolobus und ein hydrolysiertes Galactomannan umfaßt.

**2.** Zusammensetzung gemäß Anspruch 1, worin das Gewichtsverhältnis des Proteins der Bohne Cyamopsis tetragonolobus zu dem hydrolysierten Galactomannan (Protein der Bohne Cyamopsis tetragonolobus/hydrolysiertes Galactomannan) von 1/99 bis 1/5 ist.

**3.** Zusammensetzung gemäß Anspruch 1 oder 2, worin das durchschnittliche Molekulargewicht des hydrolysierten Galactomannans von 2.000 bis 100.000 ist.

**4.** Zusammensetzung gemäß einem der Ansprüche 1 bis 3, worin die Viskosität einer 0,5 (G/V)% wäßrigen Lösung des hydrolysierten Galactomannans bei 25°C 50 mPa·s oder weniger ist, wenn mit einem B-Typ-Viskometer bestimmt.

**5.** Nahrungs- oder Futtermittel, das die Zusammensetzung gemäß einem der Ansprüche 1 bis 4 umfaßt.

**6.** Verwendung des Nahrungsmittels gemäß Anspruch 5 zur Herstellung eines Nahrungsmittels zur spezifizierten Gesundheitsanwendung oder eines Nahrungsmittel für einen diabetischen Patienten.

**Revendications**

**1.** Composition permettant d'abaisser l'indice glycémique (valeur IG) d'un aliment ou d'un aliment pour animaux, comprenant une protéine de la graine de *Cyamopsis tetragonolobus* et un galactomannane hydrolysé.

**2.** Composition selon la revendication 1, dans laquelle le rapport pondéral entre la protéine de la graine de *Cyamopsis tetragonolobus* et le galactomannane hydrolysé (protéine de graine de *Cyamopsis tetragonolobus*/*galactomannane hydrolysé*) va de 1/99 à 1/5.

**3.** Composition selon les revendications 1 ou 2, dans laquelle le poids moléculaire moyen du galactomannane hydrolysé va de 2 000 à 100 000.

**4.** Composition selon l'une quelconque des revendications 1 à 3 dans laquelle la viscosité d'une solution aqueuse à 0,5 % (p/v) du galactomannane hydrolysé est inférieure ou égale à 50 mPa.s à 25 °C lorsqu'elle est déterminée avec un viscosimètre de type B.

**5.** Aliment ou aliment pour animaux comprenant la composition telle que définie dans l'une quelconque des revendications 1 à 4.

**6.** Utilisation de l'aliment selon la revendication 5 pour la fabrication d'un aliment destiné à un usage de santé spécifique ou d'un aliment destiné à un patient diabétique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 05117156 B

- JP 5117156 A

**Non-patent literature cited in the description**

- **JENKINS DJ ; GHAFARI H ; WOLEVER TM ; TAYLOR RH ; JENKINS AL ; BARKER HM ; FIELDEN H ; BOWLING AC.** Relationship between rate of digestion of foods and post-prandial glycaemia. *Diabetologia,* 1982, vol. 22, 450-455
- **HEATON KW ; MARCUS SN ; EMMETT PM ; BOLTON CH.** Particle size of wheat, maize, and oat test meals: effects on plasma glucose and insulin responses and on the rate of starch digestion in vitro. *Am. J. Clin. Nutr.,* 1988, vol. 47, 675-682
- **KUNIHIRO DOI ; KEISUKE TSUJI.** Shokumotsu Sen-i. Asakura-shoten, 1997, 412-420
- *Kagaku to Seibutsu,* 1980, vol. 18, 95-105

- **CHIU SS ; TRACK NS.** *Metabolism,* May 1985, vol. 34 (5), 481-485
- SUNFIBER. Taiyo Kagaku Co., Ltd,
- Fiberon. DAINIPPON PHARMACEUTICAL CO., LTD,
- Guar Fiber. MEIJI SEIKA KAISHA, LTD,
- G-Fiber. Glico Foods Co., Ltd,
- **WIESER H ; ANTES S ; SEILMEIER W.** *Cereal Chem.,* 1998, vol. 75, 644-650
- **GIBSON TS ; SALAH VA ; MCCLEARY BV.** *J. Cereal Sci.,* 1997, vol. 25, 111-119
- **H IWATA ; A ISOGAI ; H UTSUNOMIYA ; T ITANI ; N NISHIO.** *Nippon Nougeikagaku Kaishi,* 2003, vol. 77, 1130-1136